(19) **Europäisches Patentamt**
**European Patent Office**
**Office européen des brevets**

(11) **EP 2 613 152 A2**

(12) **EUROPEAN PATENT APPLICATION**
published in accordance with Art. 153(4) EPC

(43) Date of publication:
**10.07.2013 Bulletin 2013/28**

(51) Int Cl.:
**G01N 33/574** (2006.01)  **G01N 33/556** (2006.01)
**G01N 33/68** (2006.01)  **C07K 1/16** (2006.01)
**A61K 38/02** (2006.01)

(21) Application number: **11822200.9**

(22) Date of filing: **29.08.2011**

(86) International application number:
**PCT/RU2011/000655**

(87) International publication number:
**WO 2012/030256 (08.03.2012 Gazette 2012/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2010  RU 2010136090**

(71) Applicants:
• **Baljura, Alexandr Vladimirovich**
  **Moscow 125319 (RU)**
• **Molodyk, Alvina Alfredovna**
  **Moscow 105118 (RU)**
• **Tarasov, Sergey Gennadevich**
  **Moscow 109457 (RU)**

(72) Inventors:
• **SAVLUCHYNSKAYA, Lyudmila Alexandrovna**
  **Moscow 119311 (RU)**
• **BALYURA, Alexandr Vladimirovich**
  **Moscow 125319 (RU)**
• **MOLODYK, Alvina Alfredovna**
  **Moscow 105118 (RU)**
• **TARASOV, Sergey Gennadevich**
  **Moscow 119311 (RU)**

(74) Representative: **Isarpatent**
**Patent- und Rechtsanwälte**
**Friedrichstrasse 31**
**80801 München (DE)**

(54) **SENSITIZER FOR AN ERYTHROCYTIC DIAGNOSTIC AGENT FOR DIAGNOSING MALIGNANT NEOPLASMS AND METHOD FOR PRODUCING SAME, ERYTHROCYTIC DIAGNOSTIC AGENT FOR DIAGNOSING MALIGNANT NEOPLASMS AND METHOD FOR PRODUCING SAME AND METHOD FOR DIAGNOSING THE PRESENCE OF MALIGNANT NEOPLASMS USING SAID ERYTHROCYTIC DIAGNOSTIC AGENT**

(57)  The invention relates to medicine and applies to the diagnostics of oncological diseases, and more particularly, to the malignant neoplasms diagnostics. The sensitin for erythrocytic diagnosticum for malignant neoplasms diagnostics, as the mixture of fractions of low-molecular protein compounds with the molecular weight of 3000-15000 D, separated from serum of pregnant mares at the early stage of pregnancy, demonstrating the stimulating activity relative to the proliferation of malignant cells, is provided. The method of producing this sensitin on the basis of the pregnant mare serum is pro-

vided, as well. The sensitin-sensitized erythrocytic diagnosticum and the method of producing it are provided, as well. The method of the malignant neoplasms diagnostics using the said erythrocytic diagnosticum is provided. The claimed invention enables to diagnose the patient malignant neoplasms to a high degree of accuracy and to conduct the sufficiently fast medical examination of large population groups to diagnose the persons with malignant neoplasms.

EP 2 613 152 A2

## Description

### Field of the invention

The invention relates to medicine, and more particularly, to oncology, and it applies to diagnostics of oncological diseases.

### Background

Currently many methods of diagnostics of oncological diseases, where different erythrocytic diagnosticums are used for diagnostics of malignant neoplasms and reveal both the malignant neoplasms, and its localization in the human body, are known. As a rule, the erythrocytic diagnosticums, in which there are sensitin-sensitized erythrocytes on the basis of antigens, antibodies and specific proteins, are used to this effect.

For example, the RF Patent №2202801, G01N33/556 dated 20.04.2003 discloses the method of diagnostics using erythrocytic diagnosticum, comprising formalized sheep erythrocytes, sensitized by suitable antibodies, that enables to expose the specific disease to a high degree of accuracy. The above methods are highly efficient for detection of localized diseases, but if there is a need to diagnose an unlocalised disease, multiple diagnostic procedures using plenty of different erythrocytic diagnosticums, are required. The present patent discloses the method of producing an erythrocytic diagnosticum by means of the sensibilizating of sheep formalized erythrocytes using suitable antibodies.

The erythrocytic diagnosticum, comprising formalised sensitized sheep erythrocytes, in which an antigen or an antigen-protein coupled with carrier is used as sensitin (see the US Patent № 3987159, A61K35/12 G01N 33/16 dated 19.10.1976), is known. The present patent also discloses the method of producing diagnosticum, comprising the sheep blood processing for detection of erythrocytes, washing and formalization of erythrocytes, producing the erythrocyte suspension in buffer solution and the selected antigen- sensibilizating .

The method of diagnostics of the malignant neoplasms in the human body aside from its type and localization in the body (see, for example, the RF Patent № 22777242 G0133/49 dated 27.05.2006), is known. The known method for producing an active agent uses pregnant mare serum, produced on 45-100 day of pregnancy. The known method uses two test samples. The pregnant mare serum is added to the patient's blood in the first test sample. The normal mare serum is added to the patient's blood in the second test sample. Further, the erythrocyte sedimentation rate (ESR) in both test samples is measured and the first test sample ESR is compared to the second test sample ESR, and the diagnostic coefficient is calculated. If this is a cancer patient, the diagnostic coefficient exceeds the normal human diagnostic coefficient. The given method is based on the presence of different protein compounds demonstrating the activity against malignant cells and antigens producing by human organism in malignant neoplasms, in the blood serum of pregnant warm-blooded animals. Supposedly, the said property of pregnant warm-blooded animals' blood serum relates to the fact that during the pregnancy the stem cells fast differentiation occurs in the process of organogeny, as well as the fast increase in the cell number of organism systems. It results in taxis and production of different antigens and protein compounds, that should demonstrate the increased activity against malignant cells and their proteins, warm-blooded animal organism systems, that, in this case, according to the authors' opinion of the present invention, is confirmed by ESR increase in the patient blood.

However, in the known method the patient blood is exposed to the full set of pregnant mare serum protein compounds, comprising protein compounds, that demonstrate the active interaction with malignant cells and their proteins, as well as protein compounds, that demonstrate the weak interaction with malignant cells and their proteins, and protein compounds that are inactive against malignant cells and their proteins. The passive pregnant mare serum protein compounds decrease the concentration of active components in the pregnant mare serum, that impairs the effectiveness of pregnant mare serum for malignant neoplasms diagnostics.

### Disclosure of the invention

An object of the present invention is to separate protein compounds demonstrating the stimulating activity against malignant cells and their proteins, from pregnant mare serum at the early stage of pregnancy, the development (production) of sensitin for erythrocytic diagnosticum, the erythrocytic diagnosticum itself, sensitized by these protein compounds, as well as the development of the methods of producing sensitin and erythrocytic diagnosticum with this sensitin, and the method of diagnostics of malignant neoplasms, using this diagnosticum.

The solution of the above objects and other advantages are attained by providing sensitin for erythrocytic diagnosticum for malignant neoplasms diagnostics, representing the mixture of fractions of low-molecular protein compounds with the molecular weight of 3000-15000 D, separated from the pregnant mare serum at the early stage of pregnancy, demonstrating the stimulating activity against the proliferation of malignant cells.

The solution of the above objects and other advantages are also attained by providing the method of producing sensitin for erythrocytic diagnosticum for malignant neoplasms diagnostics, comprising the separation of pregnant mare blood serum at the early stage of pregnancy, fractionating the said serum using the liquid chromatography for separation of mixture of low-molecular protein compound fractions with molecular weight 3000-15000 D, demonstrating the stimulating activity against the proliferation of malignant cells.

Preferably, the high-pressure liquid chromatography, or gel-penetrating liquid chromatography, or fast-performance liquid chromatography (FPLC) is used for the separation of the said mixture of fractions of low-molecular compounds with molecular weight 3000-15000 D.

Preferably, the pregnant mare serum of mares on 20-60th day of pregnancy is used.

Besides, the obtained mixture of fractions of low-molecular compounds with molecular weight 3000-15000 D, separated from the pregnant mare serum are dialyzed relative to the distilled water and lyophilized.

The solution of the above objects and other advantages are also attained by providing erythrocytic diagnosticum for malignant neoplasms diagnostics, comprising formalized sheep erythrocytes, sensitized by fractions of low-molecular protein compounds with molecular weight 3000-15000 D, separated from serum of pregnant mares at early stages of pregnancy, and interacting with proteins, stimulating the proliferation of malignant cells.

Meanwhile, the said sensibilized mixture of fractions of low-molecular proteins with molecular weight 3000-15000 D, separated from serum of pregnant mares on the 20-60th day of pregnancy, is used as the said sensibilized mixture of fractions.

Meanwhile, the interaction of the said sensibilized mixture of fractions with blood serum results in the hemagglutination of the patient blood serum proteins.

The solution of the above objects and other advantages are also attained by providing the method of producing erythrocytic diagnosticum for malignant neoplasms diagnostics, comprising the sensibilizating of suspension of mammalian formalized erythrocytes in buffer solution with pH 6,4-6,6 sensitin, distinguished by the fact, that the mixture of fractions of low-molecular protein compounds with molecular weight 3000-15000 D, separated from serum of pregnant mares at early stages of pregnancy, and interacting with proteins, stimulating the proliferation of malignant cells, is used here as sensitin .

Meanwhile, the suspension of washed sheep formalized erythrocytes in phosphate buffer is used.

Besides, the 0, 15 M phosphate buffer is used for preparation of the said suspension.

Preferably, the mixture of fractions of low-molecular protein compounds with the molecular weight of 3000-15000 D, separated from serum of pregnant mares on 20-60th day of pregnancy, is used.

Preferably, the ingredients are taken in proportion: 0, 1 mg said freeze-dried sensitin to 1 ml 4% suspension of washed sheep formalized erythrocytes in phosphate buffer.

Besides, the sensitized sheep erythrocytes are stored in a refrigerator at the temperature as 2% suspension in phosphate buffer.

The solution of the above objects and other advantages are also attained by providing the method of malignant neoplasms diagnostics using the erythrocytic diagnosticum, comprising the separation of patient blood serum, preparing several patient blood serum test samples of equal volume, the first test sample comprises the patient blood whole serum, and the further test samples contain the diluted patient blood serum, and every further test sample has a lower concentration of patient blood serum, adding the equal amount of erythrocytic diagnosticum, comprising sensitin-sensitized erythrocytes, to each patient blood serum test sample, and the mixture of fractions of low-molecular protein compounds with the molecular weight of 3000-15000 D, separated from the serum of pregnant mares at early stages of pregnancy, and interacting with proteins, stimulating the proliferation of malignant cells, with further soaking of these test samples at room temperature until the completion of hemagglutination reaction, is used as sensitin, the identificating of patient blood serum test samples, where the hemagglutination reaction occurred, the patient blood serum test samples, where there was no hemagglutination reaction, the determination of minimal concentration of patient's blood serum in the test sample, when the hemagglutination reaction occurred , and/or the maximal concentration of patient's blood serum in the test sample, at which there was no hemagglutination reaction, the comparison of the results of determination of the said minimal and/or the maximal concentration of the patient's blood serum in the test sample with their threshold values, obtained for blood serum of healthy persons without malignant neoplasms, and the diagnostics of malignant neoplasms for a patient, if the results of determination of the said minimal and/or maximal concentration of patient's blood serum are worse then the said threshold values.

Meanwhile, microplates with wells, where one patient blood serum test sample is placed in each well, are used for hemagglutination reaction.

Besides, to obtain the threshold values of blood serum maximal and/or minimal concentration, the blood serum test samples of, at least, one healthy person, prepared similar to patient blood serum test samples, are placed in free wells of the same microplate, and both the minimal concentration of the normal person blood serum for these test samples, at which the hemagglutination reaction occurred is determined, and/or the maximal concentration of the normal person blood serum, at which there was no hemagglutination reaction, and these values are used as the said threshold values.

Meanwhile, the obtained threshold values of blood serum maximal and/or minimal concentration are stored.

As a variant for obtaining threshold values of blood serum maximal and/or minimal concentration, blood serum test samples are prepared for, at least, one healthy person, the hemagglutination rection occurs for these samples, the minimal concentration of the healthy person blood serum, at which the hemagglutination reaction occurs, is determined, and/or the maximal concentration of the normal blood serum, at which there was no hemagglutination reaction, and the obtained threshold values are stored.

Preferably, the erythrocytic diagnosticum is added to the the test samples as 0,16% suspension of sheep sensitized erythrocytes in the phosphate buffer with a pH of 6,4-6,6 in proportion: 10 mcl of the said suspension to 100 mcl of a test sample.

The present invention enables, using the erythrocytic diagnosticum, in which erythrocytes are sensitized by the mixture of fractions of low-molecular proteins with molecular weight 3000-15000 D, separated from the pregnant mare serum at the early stage of pregnancy, to determine the patient blood serum minimal concentration, at which the hemagglutination reaction occurred, and/or the patient blood serum maximal concentration, at which there was no hemagglutination reaction, and to diagnose the patient malignant sate to a high degree of accuracy. Meanwhile, the present invention enables to carry on an outpatient examination straight domiciliary, as the proposed erythrocytic diagnosticum can be prepared in specialized clinics and delivered to any local clinic, and the patient blood serum can be obtained straight on-site.

The present invention enables to determine quantitatively (on the basis of the patient blood serum test sample dilution coefficient) an oncologicl disease probable stage that enables to plan the program of the further examination of a patient - cancer suspect.

The present invention enables to carry out the fast examination of large groups of pupolation, so that to identify the persons with potential malignant neoplasms, irrespective of the neoplasm localization in human organism.

## DRAWINGS SHORT DESCRIPTION

It is believed, that the application drawings are clear enough for ones skilled in the art (oncology), and that these drawings are, therefore, to be construed as merely illustrative and not limitative of the present disclosure in any way whatsoever.

Fig.1 shows the chromatogram of separation of pregnant mare serum into fractions using high-pressure liquid chromatography.

Fig. 2 shows the chromatogram of separation of pregnant mare serum into fractions using the gel-penetrating liquid chromatography.

Fig. 3 shows the chromatogram of separation of pregnant mare serum into fractions using the fast-performance liquid chromatography (FPLC).

Fig. 4 shows one of microplates with blood serum test samples of healthy persons (under rows) and the patient's blood serum (lower rows) after interaction with the provided erythrocytic diagnosticum.

## SAMPLES OF EMBODIMENTS OF INVENTION

It will be appreciated, that the given description serves to illustrate the embodiments of invention, and is, therefore, to be construed as merely illustrative and not limitative of the present disclosure in any way whatsoever.

Further the possibility to obtain the active agent showing the activity against the stimulation of the proliferation of malignant cells, from the pregnant mare serum at the early stage of pregnancy that can be use as sensitin for erythrocytic diagnosticum for malignant neoplasms diagnostics, will be proved. In the description by the indication of ingredient percentage it is implied, that it is a matter of volume percents, unless otherwise specified.

To obtain the serum, the pregnant mare blood on 20-60th day of pregnancy was used. The pregnant mare blood serum was obtained using any known methods. To store the serum, 1% merthiolate was added in serum in proportion: 5ml merthiolate to 100 ml serum. The obtained serum should contain not less than 10-15% proteins and peptides.

In accordance to the first example of obtaining the said active agent, the high-pressure liquid chromatography (HPLC) is performed to determine the obtained pregnant mare serum protein saturation and to divide the pregnant mare serum proteins according to molecular weight. The Ultrapac TSK column, G 3000 SW 0.75x60 cm, "LKB", giving the division of proteins in the range of 2-340 cd, was used. The division into separate fractions was made using 0,15M phosphate buffer with the pH of 6,4-6,6 (KHPO4 + NaHPO4 + NaCl). The pregnant mare serum dose got balanced by the phosphate buffer with the same composition as the one that was used in the column. The balanced pregnant mare serum dose was added into the column and divided into fractions at the reaction rate of 12 ml/h. The pregnant mare serum separated fractions were collected in collector vials. 30 vials were used in the present test. The pregnant mare serum division showed that the examined pregnant mare serum contains an ample quantity of fractions of protein compounds in the wide range of fluctuations in their molecular weight from 200 kD ДО 3 kD with the pronounced four high peaks: the first peak is in the range of 35-45 κD, the second peak is in the range of 25 κD, the third peak is in the range of 15 kD and the fourth peak is in the range of 5 kD (see the chromatogram in Fig. 1). For each collector vial the separated mixtures of fractions of pregnant mare serum protein compounds were dialyzed relative to relative to buffer and lyophilized. 30 samples of mixture of pregnant mare protein compound fractions (according to the number of collector vials) were obtained, and those samples were tested for their activity against malignant cells proliferation.

The test of the activity of fractions of pregnant mare serum protein compounds was carried out using a MTT-test (microtetrazolium test). MTT-test is based of the enzyme recovery of tetrazolium colorless salt (3-4,5- dimethylazol-2-il)-2,5- diphenyl -2H- tetrazolium bromide) in living metabolically active cells with the formation of formazan blue cristalls .

The preliminary activity test of separated fractions of pregnant mare serum protein compounds showed, that mixtures of fractions of pregnant mare serum low-molecular protein compounds with the molecular weight of 3000D - 15000 D (corresponding to the second and the third peaks in the chromatogram, Fig.1) demonstrated the activity against stimulating the proliferation of malignant cells. Meanwhile, the fractions of low-molecular protein compounds with the molecular weight of 3000-15000 D, separated from the pregnant mare blood on the 20-60th day of pregnancy, showed the highest activity. The said mixtures of fractions of pregnant mare serum low-molecular protein compounds, having the molecular weight of 3000-15000 D, were tested for stimulating activity relative to malignant cells for three oncological diseases. The outcomes of the test for stimulating activity will be given in the further sections of description.

High molecular fractions of pregnant mare serum protein compounds, corresponding to the first and the second peaks in the chromatogram, Fig. 1, didn't show the stimulating activity relative to the proliferation of malignant cells, and those fractions weren't used in further scientific research.

In accordance to the second example of obtaining the active agent, the pregnant mare serum on the 20-60th day of pregnancy is separated into fractions using the gel-penetrating liquid chromatography. In vitro the gel-filtration of this serum in the column 100x1cm, filled with Sephadex G-150 or Sephadex G-50 was performed to separate the fractions of low-molecular protein compounds (that are of interest for us) from the pregnant mare serum. The column is balanced by 0.01M Tris HCL buffer with pH of 8.0. The pregnant mare serum dose got balanced by the buffer of the same composition that was used in the column. The balanced pregnant mare serum dose was placed in the column and separated into fractions at the reaction rate of 12 ml/h or 30-40 ml/h. The separated fractions of pregnant mare serum were collected in collector vials. In the present test 48 collector vials were used. The chromatogram of pregnant mare serum, obtained using the gel-filtration of the said serum in the chromatographic column, is shown in Fig. 2. The chromatogram in Fig. 2 coincides with the chromatogram in Fig.1, and also shows the high peak for fractions of pregnant mare serum low-molecular protein compounds with molecular weight of 3000-1500 D, that are collected in 15, 16 and 17 collector vials. Several gel-filtrations are performed to get the sufficient amount of the test mixture of fractions of pregnant mare serum low-molecular protein compounds, and the content of a specified control vial is summarized. The separated mixtures of fractions of pregnant mare serum protein compounds are dialyzed against the buffer solution and liophilized. 48 liophilized samples were collected and their stimulating activity test using MTT-test (microtetrazolium test) was performed. In the said test the outcomes of the stimulating activity estimate of protein compound fractions coincide with the outcomes obtained for the samples of the same fractions on the basis of the high-pressure liquid chromatography method. The fractions of pregnant mare low-molecular protein compounds from 13-17 collector vials were used for further research of stimulating activity.

In accordance to the third examples of obtaining the said active agent, the pregnant mare serum on the 20-60th day of pregnancy is divided into fractions using fast-performance liquid chromatography (FPLC) in column mono Q using two buffers A and B. The pregnant mare serum chromatogram, obtained using the fast-performance liquid chromatography (FPLC), is shown in Fig. 3. The chromatogram in Fig. 3 coincides with the chromatogram in Fig.1, and also shows the high peak for fractions of pregnant mare serum low-molecular protein compounds with molecular weight of 3000-15000 D, that are collected in 15, 16 and 17 collector vials. Using the said method, the mixture of fractions of pregnant mare serum low-molecular protein compounds with molecular weight 3000-15000 D, was separated, and the lyophilized samples of the tested fractions of pregnant mare serum low-molecular protein compounds were prepared on the basis of these fractions.

**The stimulative activity test for the malignant cells' proliferation of fractions of pregnant mare serum low-molecular proteins, meaning the mares at the early stage of pregnancy.**

The activity test of fractions of pregnant mare serum protein compounds is performed using MTT-test (microtetrazolium test). MTT-test is based on the enzyme recovery of tetrazolium colorless salt (3-4,5- dimethylazol-2-il)-2,5-diphenyl -2H-tetrazolium bromide) in living metabolically active cells with the formation of formazan blue cristalls.

The microplates with 96 wells are used for tests. The culture of cancer cells - 17 thousand cells in 5% $CO_2$ -

was placed in each cell. Then the tested fractions of pregnant mare serum protein compounds in the given concentration - 20 micrograms of tested mixtures of fractions in 180 mcl of distilled water (dilution 1:10) - were added in wells and are incubated during 48 hours under the same conditions. Upon the complition of the incubation, the MTT-reagent with the final concentration of 25 mcg/ml was added in a well and then incubated within 4 hours under the same conditions. The formed formazan blue cristalls were dissolved in dimethyl sulfoxide (DMSO) within 20 minutes. The optical absorption was measured using the Titertek Multiskan MCC/340 spectrophotometer at the wave length 570 nm. The outcomes obtained for tested samples of fractions of pregnant mare serum protein compounds were compared with the outcomes of control samples (without adding the tested fractions of pregnant mare serum protein compounds to cell culture). To assess the stimulating activity of fractions of pregnant mare serum protein compounds on cells, the CK coefficient, calculated on the basis of the known formula, was used:

$$CK\% = (1\text{-}N \text{ test}/N \text{ control}) \times 100\%.,$$

meaning the formula, used for quantitative assessment of active agent stimulating activity.

The stimulating effect of mixtures of fractions of serum low-molecular compounds of pregnant mares at early stages of pregnancy relative to malignant cells, is tested for three oncological diseases: human colon cancer, human cancer of lung and human ovarian carcinoma.

**Test 1**

In this set of tests the effect of fractions of low-molecular pregnant mare serum protein compounds with the molecular weight of 3000-15000 D on the culture of malignant cells of human lung cancer SKOV-3, was checked. The tests were performed using MTT-test (microtetrazolium test) in accordance to the above-mentioned technique. The test results are shown in the Table 1. The buffer (tris-buffer) was used as a control sample.

Table №1

| № fractions (№ collector vial) | Result of interaction |
|---|---|
| Control buffer(tris-buffer) | Inhibition 2,0% |
| Fraction №13 | No effect |
| Fraction №14 | No effect |
| Fraction №15 | Stimulation 18% |
| Fraction №16 | Stimulation 32% |
| Fraction №17 | Stimulation 30% |

As it is seen from the Table 1, the fractions of blood serum low-molecular proteins, collected in the control vials 15, 16 and 17 and having the molecular weight of 3000-15000 D, demonstrate the pronounced stimulating effect relative to the proliferation of human ovarian carcinoma malignant cells SKOV-3.

**Test 2**

In this set of tests, the effect of fractions of pregnant mare serum low-molecular protein compounds with the molecular weight of 3000-15000 D on the culture of human colon cancer malignant cells was tested. The tests were conducted using MTT-test (microtetrazolium test) in accordance to the above-mentioned technique. The test results are shown in the Table 2. The buffer (tris-buffer) is used as a control sample.

Table №2

| № fractions (№ collector vial) | Result of interaction |
|---|---|
| Control buffer(tris-buffer) | Inhibition 5,0% |
| Fraction №13 | No effect |
| Fraction №14 | No effect |
| Fraction №15 | Stimulation 22% |

(continued)

| № fractions (№ collector vial) | Result of interaction |
|---|---|
| Fraction №16 | Stimulation 41% |
| Fraction №17 | Stimulation 28% |

As it is seen from the Table 2, the fractions of blood serum of low-molecular proteins, collected in the control vials 15, 16 and 17 and having the molecular weight of 3000-15000 D, demonstrate a pronounced stimulating effect relative to the proliferation of human colon cancer malignant cells.

**Test 3**

In this set of tests, the effect of fractions of pregnant mare serum low-molecular protein compounds with the molecular weight of 3000-15000 D on the culture of human lung cancer malignant cells , was tested. The tests were conducted using MTT-test (microtetrazolium test) in accordance to the above-mentioned technique. The test results are shown in the Table 3. The buffer (tris-buffer) is used as a control sample.

Table №3

| № fractions (№ collector vial) | Result of interaction |
|---|---|
| Control buffer(tris-buffer) | Inhibition 7,0% |
| Fraction №13 | No effect |
| Fraction №14 | No effect |
| Fraction №15 | Stimulation 16% |
| Fraction №16 | Stimulation 26% |
| Fraction №17 | Stimulation 22% |

As it is seen from the Table 3, the fractions of blood serum low-molecular proteins, collected in the control vials 15, 16 and 17 and having the molecular weight of 3000-15000 D, demonstrate a pronounced stimulating effect relative to the proliferation of malignant cells of human cancer of lung.

As it is seen from the results of tests 1-3, the fractions of pregnant mare serum low-molecular proteins demonstrate a pronounced stimulating effect relative to the proliferation of malignant cells of different oncological diseases. The minimal observable CK coefficient was 16% that, as it will be shown further, is sufficient for usage of these fractions in erythrocytic diagnosticum for malignant neoplasms diagnostics.

Thus, the proposed method of producing active agent from the pregnant mare serum at the early stage of pregnancy, provides the obtaining of sensitin, demonstrating the stimulating activity relative to malignant cells, for sensibilizating of diagnosticum erythrocytes for malignant neoplasms diagnostics.

To use the revealed active agent, that is the mixture of fractions of low-molecular protein compounds with molecular weight of 3000-15000 D, separated from pregnant mare serum at the early stage of pregnancy, the erythrocytic diagnosticum is proposed, where the said active agent is used as sensitin.

The proposed erythrocytic diagnosticum contains formalised sheep erythrocytes, loaded with sensitin (active agent), demonstrating the stimulating activity relative to malignant cells. The mixture of fractions of low-molecular compounds with molecular weight of 3000-15000 D, separated from a pregnant mare serum at the early stage of pregnancy, is used as sensitin. In the reaction with sick people's blood serum the proposed erythrocytic diagnosticum can identify the presence and activity of binding proteins, participating in the process of malignant transformation.

The proposed erythrocytic diagnosticum is produced as follows.

The sheep blood, taken in the Alsever's solution, is exposed to the formalinization using any known method. In the claimed invention the sheep blood was hold in the refrigerator during 3-4 days for stabilization of erythrocytes, then the washing and formalinization of sheep erythrocytes were performed. The washed formalised sheep erythrocytes can be used for obtaining erythrocytic diagnosticum directly after their preparation, or they could be stored for further usage in the refrigerator as 2% suspension of erythrocytes in distilled water.

For sensibilizating (loading) of sheep erythrocytes, the 4% suspension of washed sheep formalized erythrocytes in 0,15 M phosphate buffer with pH of 6,4-6,6, similar to buffer, used in chromatographic columns in the process of separation of active agent from pregnant mare serum, is prepared. During the sensibilizating of sheep erythrocytes, the mixture of fractions of low-molecular protein compounds with molecular weight 3000-15000 D, separated using the chromatograph from the serum of pregnant mares at early stages of pregnancy, preferably on the 20-60th day of pregnancy, in proportion: 0,1 mg lyophilized fractions of pregnant mare serum low-molecular protein compounds to 1 ml 4% suspension of washed sheep formalized erythrocytes in phosphate buffer, is added in the 4% suspension of sheep erythrocytes. The process of erythrocyte sensibilizating occurs in an incubator at the temperature 45- 56C. The said fractions demonstrate the stimulating activity relative to malignant cells, and interact with proteins, stimulating the proliferation of malignant cells, that enables to use this erythrocytic diagnosticum for patient's malignant neoplasms diagnostics without localization of malignant neoplasms in the patient organism actually at any stage of carcinogenesis.

The proposed erythrocytic diagnosticum can be used directly after its preparation. In case of necessity to store the erythrocytic diagnosticum for further usage, 4% suspension of washed sheep formalized erythrocytes in phosphate buffer, that is stored in a refrigerator, is prepared from 2% suspension of washed sheep formalized erythrocytes in phosphate buffer.

The method of patient's malignant neoplasms diagnostics is implemented as follows.

The patient's malignant neoplasms diagnostics is performed on micropates having 8 wells with the capacity of 250 mcl in every horizontal row, but other microplates can be used as well.

In the course of the patient's examination for presence (or absence) of neoplasms, the patient blood test, for example, in the volume of 3,5-6 ml., is taken and separated from serum. The obtained serum is divided into several doses and serum test samples are prepared, meanwhile the first test sample contains the patient's whole serum blood, and the further test samples contain the diluted phosphate buffer, similar the buffer, used in the process of producing the erythrocytic diagnosticum, the patient blood serum, and each further test sample has lower serum concentration. In the said test the serum concentration decreases twice as much in every further test samples , as in the said test the dilution coefficient increment is 2, but it is possible to prepare the test samples with any acceptable dilution coefficient increment, for example, 1,5 or 2,5. Every patient blood serum test sample, for example, in the amount of 100 mcl, is placed in the relevant well of microplates, so that in the consecutive row of wells with tested serum sample, the serum concentration in wells decreases in proportion to the dilution coefficient increment: in the first well - 100% patient blood serum, in the second well - 50%, and in the last tenth well - the concentration serum is in 512 times less, than in the first well. In case of the large amount of wells, or if there is the other coefficient increment, it is possible to get a test sample, in which the blood serum concentration is in thousands or several thousands times less, then in the first well.

For immediate diagnostics of the patient's blood serum said sample for malignant neoplasms from the early-prepared erythrocytic diagnosticum, the 0,16% suspension of sheep erythrocytes, sensitized by pregnant mare serum low molecular proteins with the molecular weight of 3000-15000 D, demonstrating the stimulating activity relative to malignant cells, in the phosphate buffer solution. In each well with a patient's blood serum, 10 mcl 0, 16% suspension of sheep erythrocytes is added. Thus, the equal amount of active ingredient (sensitized on erythrocyte of low-molecular proteins, separated from the pregnant mare serum) is provided in each well.

The blood serum test samples of healthy persons, prepared in accordance to the above-mentioned technique, and placed in wells in one of the horizontal rows of microplates, are used as a control sample.

The microplate with loaded wells is shaked carefully and hold at room temperature and incubated at room temperature within 2,5 - 8 hours, so that in test samples the passive hemagglutination reaction occures, and then it is identified visually in which wells the hemagglutination reaction occurs, and in which wells the hemagglutination reaction is not registered . In the well, where the hemagglutination reaction occurred in the test sample, the solution grows turbid. In the well, in which the hemagglutination reaction doesn't occur in the test sample, the solution remains clear and erythrocytes settle on the well bottom. For each well it is known, which patient's blood serum test sample is located in this well, and, correspondingly, the patient's blood serum concentration (dilution coefficient) in the said well, is known. Knowing the patient blood serum dilution degree for the said patient blood serum test sample, it is possible to determine at which patient's blood serum dilution degree the hemagglutination reaction occured and at which patient's blood serum dilution degree the hemagglutination reaction terminates. These data are used for patient's malignant neoplasms diagnostics without the malignant neoplasms localization in human organism.

Fig. 4 shows one of microplates with blood serum test samples of healthy people (upper rows) and patient's blood serum (lower rows) after interaction with proposed erythrocytic diagnosticum.

The proposed technique of malignant neoplasms diagnostics with successive dilution of patient's blood serum enables to obtain the quantitative characteristic of interaction of erythrocytic diagnosticum active ingredient with its binding proteins and patient blood serum receptors. The higher is a patient's serum dilution, initiating the agglutination reaction, the higher is the content of binding factors in the patient blood binding serum, and the higher is the proliferation degree of malignant cells. To increase the accuracy of malignant neoplasms diagnostics, it is reasonable to use the test samples with lower dilution coefficient along with the quantitative characteristic of the proliferation degree of malignant cells.

The tests, where the normal human blood serum is used, enable to obtain the quantitative characteristic of the proposed erythrocytic test activity relative to the normal blood serum and use these results as basic level for malignant neoplasms diagnostics.

In accordance to the above-mentioned technique the tests for malignant neoplasms diagnostics for several groups of patients with different localizations of malignant neoplasms are performed. One of patient groups participated in each set of tests.

**Test 4.**

In this set of tests the data on interaction of proposed erythrocytic diagnosticum with serum of healthy people, are collected. 27 persons participated in the test. For each person the set of 10 blood serum test samples with volume of 100 mcl was prepared, and the patient serum concentration in test samples varied as follows: the whole blood serum in the first test sample (dilution coefficient 1:1) and in each successive test sample the blood serum concentration decreases twice as much, and the tenth test sample had the dilution coefficient of 1:512. 10 mcl proposed erythrocytic diagnosticum was added in each test sample. While testing, the wells, in which the hemagglutination reaction occurred, were registered visually, on the basis of solution dimness, and the last well of this set, in which the hemagglutination reaction occurred, was determined, and correspondingly, the minimal serum concentration, at which the hemagglutination reaction occurred (blood serum dilution coefficient in the said well), was determined, as well. The blood serum concentration in the successive well, in which the solution remained clear and erythrocytes settled on the well bottom , showed at which maximal blood serum concentration the hemagglutination reaction didn't occur. The test results are given in Table 1, where it is shown the following: the number of persons in the test (set of test samples), minimal blood serum concentration (dilution coefficient), at which the hemagglutination reaction occurred in the well, and a number of wells in the set, meaning the wells, in which the hemagglutination reaction occured at the said minimal blood serum concentration.

Table 4.

| Number of persons in the group | 57 |
| --- | --- |
| Minimal patient blood serum concentration, at which the hemagglutination reaction occured | Number of wells with the said minimal patient blood serum concentration, at which the hemagglutination reaction occured |
| 1:4 | |
| 1:8 | |
| 1:16 | |
| 1:32 | 6 |
| 1:64 | 0 |
| 1:128 | 0 |
| 1:256 | 0 |
| 1:512 | 0 |

The tests for interaction of proposed erythrocytic diagnosticum with blood serum of healthy people showed, that proposed erythrocytic diagnosticum demonstrates the high activity relative to the human blood serum and interacts actively both with the normal whole blood serum, and with the diluted normal blood serum at the dilution coefficients 1: 2, 1:4, 1:8 and 1:16.

As it is seen from Table 4 with pretty high degree of accuracy (90%), the blood serum concentration 1:16 can be taken as the threshold value of patient's blood serum concentration in the course of the malignant neoplasms diagnostics, but the patient blood serum threshold value during the malignant neoplasms diagnostics can be obtained simultaneously with the diagnostic process, placing the test samples with normal blood serum in microplate wells, to obtain the threshold value directly for the specified situation.

Further the data on malignant neoplasms diagnostics for patients with different localization of malignant neoplasms are given. In the following examples the threshold values, obtained in the test 4, are used, but one should understand, that the said example doesn't reduce the proposed diagnostics method only to this case, and it is possible to use the blood serum concentration threshold value, obtained directly in the course of hemagglutination reaction with the patient blood serum.

**Test 5**

In this set of tests the group of patients with breast cancer, 54 in number, was examined. The examination technique coincides with the above-mentioned one, described in the Test 4. The test results are given in Table 5, where the number of persons in the test (the set of test samples), the minimal blood serum concentration (dilution coefficient), at which the hemagglutination reaction occurred in the well, and the number of wells in the set, in which the hemagglutination reaction occurred at the said minimal blood serum concentration, are shown.

Table 5.

| Number of persons in the group | 54 |
| --- | --- |
| Minimal patient blood serum concentration, at which the hemagglutination reaction occured | Number of wells with the said minimal patient blood serum concentration, at which the hemagglutination reaction occured |
| 1:16 | 5 |
| 1:32 | 5 |
| 1:64 | 10 |
| 1:128 | 22 |
| 1:256 | 12 |
| 1:512 | 0 |

As it is seen from Table 5, if the minimal concentration 1:16 is taken as the threshold value (as it is determined in Test 4), the accuracy of diagnostics of breast cancer malignant cells is about 90%.

**Test 6**

In this set of tests the group of patients with ovarian carcinoma, 61 in number, was examined. The examination technique coincides with above-mentioned one in the Test 4. Test results are given in the Table 6, where the number of persons in the test (the set of test samples), the minimal blood serum concentration (dilution coefficient), at which the hemagglutination reaction occurred in the well, and the number of wells in the set, in which the hemagglutination reaction occurred at the said minimal blood serum concentration, are shown.

Table 6.

| Number of persons in the group | 61 |
| --- | --- |
| Minimal patient blood serum concentration, at which the hemagglutination reaction occured | Number of wells with the said minimal patient blood serum concentration, at which the hemagglutination reaction occured |
| 1:16 | 3 |
| 1:32 | 20 |
| 1:64 | 18 |
| 1:128 | 9 |
| 1:256 | 8 |
| 1:512 | 3 |

As it is seen from Table 6, if the minimal concentration 1:16 is taken as the threshold value (as it is determined in Test 4), the accuracy of diagnostics of ovarian carcinoma malignant cells is about 96%.

**Test 7**

In this set of tests the group of patients with stomach cancer, 33 in number, was examined. The examination technique coincides with above-mentioned one in the Test 4. Test results are given in the Table 7, where the number of persons in the test (the set of test samples), the minimal blood serum concentration (dilution coefficient), at which the hemagglutination reaction occurred in the well, and the number of wells in the set, in which the hemagglutination reaction occurred at the said minimal blood serum concentration, are shown.

Table 7.

| Number of persons in the group | 33 |
|---|---|
| Minimal patient blood serum concentration, at which the hemagglutination reaction occured | Number of wells with the said minimal patient blood serum concentration, at which the hemagglutination reaction occured |
| 1:16 | 1 |
| 1:32 | 7 |
| 1:64 | 5 |
| 1:128 | 12 |
| 1:256 | 3 |
| 1:512 | 5 |

As it is seen from Table 7, if the minimal concentration 1:16 is taken as the threshold value (as it is determined in Test 4), the accuracy of diagnostics of stomach cancer malignant cells is about 94%.

**Test 8**

In this set of tests the group of patients with uterine cancer, 33 in number, was examined. The examination technique coincides with the above-mentioned one in the Test 4. The test results are given in the Table 8, where the number of persons in the test (the set of test samples), the minimal blood serum concentration (dilution coefficient), at which the hemagglutination reaction occurred in the well, and the number of wells in the set, in which the hemagglutination reaction occurred at the said minimal blood serum concentration, are shown.

Table 8.

| Number of persons in the group | 33 |
|---|---|
| Minimal patient blood serum concentration, at which the hemagglutination reaction occured | Number of wells with the said minimal patient blood serum concentration, at which the hemagglutination reaction occured |
| 1:16 | 6 |
| 1:32 | 7 |
| 1:64 | 5 |
| 1:128 | 8 |
| 1:256 | 7 |
| 1:512 | 0 |

As it is seen from Table 8, if the minimal concentration 1:16 is taken as the threshold value (as it is determined in Test 4), the accuracy of diagnostics of uterine cancer malignant cells is about 82%.

**Test 9**

In this set of tests the group of patients with prostate cancer, 29 in number, was examined. The examination technique coincides with above-mentioned one in the Test 1. Test results are given in the Table 6, where the number of persons in the test (the set of test samples), the minimal blood serum concentration (dilution coefficient), at which the hemagglutination reaction occurred in the well, and the number of wells in the set, in which the hemagglutination reaction occurred at the said minimal blood serum concentration, are shown.

Table 9.

| Number of persons in the group | 29 |
|---|---|
| Minimal patient blood serum concentration, at which the hemagglutination reaction occured | Number of wells with the said minimal patient blood serum concentration, at which the hemagglutination reaction occured |
| 1:16 | 4 |
| 1:32 | 2 |
| 1:64 | 15 |
| 1:128 | 3 |
| 1:256 | 5 |
| 1:512 | 0 |

As it is seen from Table 9, if the minimal concentration 1:16 is taken as the threshold value (as it is determined in Test 4), the accuracy of diagnostics of prostate cancer malignant cells is about 86%.

**Test 10**

In this set of tests the group of patients with colon cancer, 63 in number, was examined. The examination technique coincides with the above-mentioned one in the Test 4. The test results are given in the Table 10, where the number of persons in the test (the set of test samples), the minimal blood serum concentration (dilution coefficient), at which the hemagglutination reaction occurred in the well, and the number of wells in the set, in which the hemagglutination reaction occurred at the said minimal blood serum concentration, are shown.

Table 10.

| Number of persons in the group | 63 |
|---|---|
| Minimal patient blood serum concentration, at which the hemagglutination reaction occured | Number of wells with the said minimal patient blood serum concentration, at which the hemagglutination reaction occured |
| 1:16 | 5 |
| 1:32 | 6 |
| 1:64 | 19 |
| 1:128 | 24 |
| 1:256 | 9 |
| 1:512 | 0 |

As it is seen from Table 10, if the minimal concentration 1:16 is taken as the threshold value (as it is determined in Test 4), the accuracy of diagnostics of colon cancer malignant cells is about 92%.

**Test 11**

In this set of tests the group of patients with thyroid carcinoma, 15 in number, was examined. The examination technique coincides with the above-mentioned one in the Test 4. The test results are given in the Table 11, where the number of persons in the test (the set of test samples), the minimal blood serum concentration (dilution coefficient), at which the hemagglutination reaction occurred in the well, and the number of wells in the set, in which the hemagglutination reaction occurred at the said minimal blood serum concentration, are shown.

Table 11.

| Number of persons in the group | 15 |
|---|---|
| Minimal patient blood serum concentration, at which the hemagglutination reaction occured | Number of wells with the said minimal patient blood serum concentration, at which the hemagglutination reaction occured |
| **1:16** | **5** |
| **1:32** | **3** |
| **1:64** | **4** |
| **1:128** | **3** |
| **1:256** | **0** |
| **1:512** | **0** |

As it is seen from Table 11, if the minimal concentration 1:16 is taken as the threshold value (as it is determined in Test 4), the accuracy of diagnostics of thyroid carcinoma malignant cells is about 66%. In case of such results, it is reasonable to repeat the examination using the threshold value determination alongside with the malignant neoplasms diagnostics.

**Test 12**

In this set of tests the group of patients with testicular carcinoma, 9 in number, was examined. The examination technique coincides with above-mentioned one in the Test 4. Test results are given in the Table 12, where the number of persons in the test (the set of test samples), the minimal blood serum concentration (dilution coefficient), at which the hemagglutination reaction occurred in the well, and the number of wells in the set, in which the hemagglutination reaction occurred at the said minimal blood serum concentration, are shown.

Table 12

| Number of persons in the group | 9 |
|---|---|
| Minimal patient blood serum | Number of wells with the said |
| **1:16** | **2** |
| concentration, at which the hemagglutination reaction occured | minimal patient blood serum concentration, at which the hemagglutination reaction occured |
| **1:32** | **2** |
| **1:64** | **1** |
| **1:128** | **4** |
| **1:256** | **0** |
| **1:512** | **0** |

As it is seen from Table 12, if the minimal concentration 1:16 is taken as the threshold value (as it is determined in Test 4), the accuracy of diagnostics of testicular carcinoma malignant cells is about 80%.
The said data demonstrate the high efficiency of the proposed erythrocytic diagnosticum for diagnostics of patient malignant neoplasms regardless of the malignant neoplasm localization.
Thus, the proposed method of producing the active agent, as the fractions of pregnant mare serum low-molecular proteins with the molecular weight of 3000-15000 D, obtained at the early stage of pregnancy, provides the obtaining the active agent, demonstrating the stimulating activity relative to the stimulation of proliferation of malignant cells, for the malignant neoplasms diagnostics, and this method can be used for obtaining the erythrocytic diagnosticum for reliable malignant s neoplasms diagnostics.
The erythrocytic diagnosticum, sensitized by the fractions of low-molecular protein compounds with the molecular weight of 3000-15000 D, separated from pregnant mare serum at the early stage of pregnancy, can be used for diagnostics of the patient malignant neoplasms regardless of the malignant neoplasm localization.

The application of the said erythrocytic diagnosticum doesn't relate to the direct impact on the human organism, that pre-determines the good prospects for the proposed erythrocytic diagnosticum and the method of the patient's malignant neoplasms diagnostics both in the "real-life" clinical practice, and in the course of the mass health examination aimed at the early diagnostics of oncological diseases.

The quantitative characteristics, given in the examples of the invention embodiments, meaning the characteristics concerning the concentration of solutions, the volume of wells and their microplate number, the patient blood serum test sample volumes, the volume added to the erythrocytic diagnosticum test volume and its concentration, describe only the example of the invention embodiment and don't limit the spirit and scope of invention.

## IINDUSTRIAL APPLICABILITY

The present invention used <u>the same</u> raw materials base, the pregnant mare blood or the pregnant mare serum, that is already used in modern medicine. The above-mentioned variant of the invention embodiment is just an example and it doesn't limit the present invention. The description of the present invention is an illustrative one and it doesn't limit the scope of the patent claims. One skilled in the art can make various changes and modifications of the invention relating to the specified embodiment thereof, without departing from the spirit and scope of the invention.

### Claims

1.　Sensitin for erythrocytic diagnosticum of malignant neoplasms diagnostics, which is the mixture of fractions of low-molecular protein compounds with the molecular weight of 3000-15000 D, separated from the pregnant mare serum at the early stage of pregnancy, demonstrating the stimulating activity relative to the proliferation of malignant cells.

2.　The method of producing sensitin for erythrocytic diagnosticum of malignant neoplasms diagnostics, comprising the obtaining of pregnant mare serum at the early stage of pregnancy, the fractionating of the said serum using liquid chromatography, so that to separate from serum the mixture of fractions of low-molecular protein compounds with molecular weight of 3000-15000 D, demonstrating the stimulating activity against the proliferation of malignant cells.

3.　The method according to the claim 2, wherein the high-pressure liquid chromatography is used for the separation of the said mixture of the fractions of low-molecular compounds with the molecular weight of 3000-15000 D .

4.　The method according to the claim 2, wherein the gel-penetrating liquid chromatography is used for the separation of the said mixture of fractions of low-molecular compounds with the molecular weight of 3000-15000 D.

5.　The method according to the claim 2, wherein the fast-performance liquid chromatography (FPLC) is used for the separation of the said mixture of fractions of low-molecular compounds with the molecular weight of 3000-15000 D.

6.　The method according to the claim 2, wherein the serum pregnant on the 20-60$^{th}$ day of pregnancy, is used

7.　The method according to the claim 2, wherein the obtained mixture of fractions of low-molecular compounds with the molecular weight of 3000-15000 D, separated from the pregnant mare serum, is dialyzed relative to distilled water and liophilized.

8.　The erythrocytic diagnosticum of malignant neoplasms diagnostics, comprising formalised sheep erythrocytes, sensitized by the mixture of fractions of low-molecular protein compounds with molecular weight of 3000-15000 D, separated from the serum of pregnant mares at early stages of pregnancy, and interacting with proteins, stimulating the proliferation of malignant cells.

9.　The erythrocytic diagnosticum according to the claim 8, wherein the mixture of fractions of low-molecular proteins with the molecular weight of 3000-15000 D, separated from the serum of pregnant mares on the 20-60$^{th}$ day of pregnancy, is used as the said sensibilized mixture of fractions.

10.　The erythrocytic diagnosticum according to the claim 8, wherein the interaction of the said sensibilized mixture of fractions with blood serum results in the hemagglutination of patient blood serum proteins.

11.　The method of producing erythrocytic diagnosticum for malignant neoplasms diagnostics, comprising the sensibilizating of the suspension of formalized mammal erythrocytes in the buffer solution with the pH of 6,4-6,6 by sensitinoM, **characterized by** the fact , that the mixture of fractions of low-molecular protein compounds with the molecular weight of 3000-15000 D, separated from the serum of pregnant mares at early stages of pregnancy, and interacting with proteins, stimulating the proliferation of malignant cells, is used as sensitin.

12. The method according to the claim 11, wherein the suspension of washed formalized sheep erythrocytes in phosphate buffer, is used.

13. The method according to the claim 12, wherein 0, 15 M phosphate buffer is used for the preparation of the said suspension.

14. The method according to the claim 11, wherein the mixture of fractions of low-molecular protein compounds with the molecular weight of 3000-15000 D, separated from the mare of pregnant mares on the 20-60th day of pregnancy, is used.

15. The method according to the claim 12, wherein the ingredients are taken in proportion: the 0, 1 mg said freeze-dried sensitin to 1 ml 4% suspension of washed formalized sheep erythrocytes in phosphate buffer.

16. The method according to the claim 13, wherein the sensitized sheep erythrocytes are stored in a refrigerator at the temperature as 2% suspension in phosphate buffer.

17. The method of malignant neoplasms diagnostics using the erythrocytic diagnosticum, comprising obtaining of the patient blood serum, the preparation of several patient's blood serum test samples of equal volume, and the first test sample contains the patient's whole blood serum, and the further samples contains the patient's diluted blood serum, and each successive test sample has lower patient blood serum concentration, adding the equal amount of erythrocytic diagnosticum, comprising the sensitin-sensitized erythrocytes to each patient's blood serum test sample, wherein the mixture of fractions of low-molecular protein compounds with molecular weight of 3000-15000 D, separated from the serum of pregnant mares at early stages of pregnancy and interacting with proteins stimulating the proliferation of malignant cells is used as sensitin, further soaking of these test samples at room temperature until the stopping of the hemagglutination reaction, the identificating of patient blood serum test samples, wherein the hemagglutination reaction occurred, and the patient blood serum test samples, wherein the hemagglutination reaction didn't occur, the determination of the patient's blood serum minimal concentration in the test sample, at which the hemagglutination reaction occurred, and/or the patient's blood serum maximal concentration in the test sample, at which the hemagglutination reaction didn't occur, the comparison of the results of the said patient's blood serum minimal and/or maximal concentration in the test sample with their threshold values, obtained for normal blood serum of people without malignant neoplasms and diagnosting the presence the patient's malignant neoplasms, if the results of the said patient's blood serum minimal and/or maximal concentration are worse, then the said threshold values.

18. The method of diagnostics according to the claim 17, wherein the microplates with wells are used for the hemagglutination reaction, and one patient blood serum test sample is placed in each well.

19. The method of diagnostics according to the claim 18, wherein to obtain the threshold values of blood serum maximal and/or minimal concentration, the blood serum test samples, at least, from one healthy person, prepared similar to the patient blood serum test samples, are placed in free wells of the same microplate, and the minimal normal blood serum concentration, at which the hemagglutination reaction occurred, and/or the maximal normal blood serum concentration, at which the hemagglutination reaction didn't occur, are determined for these test samples, and these concentrations are used as the said threshold values.

20. The method of diagnostics according to the claim 19, wherein the obtained threshold values of blood serum maximal and/or minimal concentration are stored .

21. The method of diagnostics according to the claim 18, wherein to obtain the threshold values of blood serum maximal and/or minimal concentration, the blood serum test samples of, at least, one healthy person, are prepared, the hemagglutination reaction was performed for them, the minimal normal blood serum concentration, at which the hemagglutination reaction occurred, and/or the maximal normal blood serum concentration, at which the hemagglutination reaction didn't occur are determinated, and the obtained threshold values are stored.

22. The method of diagnostics according to any of claims 17-21, wherein the erythrocytic diagnosticum is added to the test samples in the form of 0, 16% suspension of sensitized sheep erythrocytes in phosphate buffer with the pH of 6,4-6,6 in proportion: 10 mcl of the said suspension to 100 mcl of the test sample.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2202801
- US 3987159 A

- FR 22777242G013349